# EUROPÄISCHE PATENTSCHRIFT

(11) **EP 2 325 089 B1**
(45) Veröffentlichungstag und Bekanntmachung des Hinweises auf die Patenterteilung: **21.12.2016**
(21) Anmeldenummer: 11154115.7
(22) Anmeldetag: 18.04.2008
(51) Int. Cl.: B65B 55/08, B67C 7/00, H01J 33/02, H01J 33/00, A61L 2/08

(54) **Vorrichtung zum Sterilisieren von Behältnissen**
Device for sterilising containers
Dispositif de stérilisation de récipients

(30) Priorität: 11.02.2008 US 27709 P; 19.04.2007 EP 07007977
(43) Veröffentlichungstag der Anmeldung: 25.05.2011
(62) Teilanmeldung aus: 08154775.4
(73) Patentinhaber: Krones AG, 93073 Neutraubling (DE)
(72) Erfinder: Humele, Heinz, 93107, Thalmassing (DE); Krüger, Jochen, 93095 Hagelstadt (DE); Menz, Hans-Jürgen, 71522, Backnang (DE); Föll, Eberhard, 72147, Nehren (DE)
(74) Vertreter: Bittner, Bernhard

(56) Entgegenhaltungen:
- EP-A- 1 120 121
- EP-A2- 1 775 752
- EP-B1- 0 054 016
- WO-A-02/36437
- WO-A-97/07024
- WO-A1-2004/013889
- WO-A2-2007/095205
- CH-A5- 651 155
- DE-A1- 10 134 037
- DE-A1- 10 236 683
- GB-A- 277 347
- GB-A- 2 271 347
- JP-U- S5 138 198
- US-A- 2 602 751
- US-A- 3 621 327
- US-B1- 6 407 492

## Beschreibung

Die vorliegende Erfindung betrifft eine Vorrichtung zum Sterilisieren von Behältnissen, genauer gesagt zum Sterilisieren von Behältnissen vor deren Abfüllung. Speziell soll durch die erfindungsgemäße Vorrichtung die Innenwandung von zu befüllenden Behältnissen, insbesondere von Flaschen, welche eine Mündung aufweisen, sterilisiert werden.

Wenn Lebensmittel in ein Behältnis eingefüllt werden, ist es erforderlich, das Behältnis selbst zu sterilisieren. So ist es beispielsweise bekannt, die Innenwandung von Behältnissen mit Dampf oder Wasserstoffperoxid zu sterilisieren. Derartige Verfahren sind jedoch mit Nachteilen verbunden, da es durch die Behandlung beispielsweise mit Wasserstoffperoxid zu einer Aufweichung des Materials kommen kann. Daher ist es aus dem Stand der Technik seit langen bekannt, die Behältnisse mit Hilfe von Ladungsträgern wie insbesondere Elektronenstrahlen zu sterilisieren.

Aus der JP 2002-255125 ist eine Vorrichtung zum Sterilisieren von Behältnissen bekannt. Dabei ist eine außerhalb des Behältnisses vorgesehene Strahlungsquelle vorgesehen, die Strahlung in das Innere des Behältnisses richtet. Auch in der JP 2001-225814 wird eine entsprechende Vorrichtung zur Innenwandsterilisation von Behältnissen beschrieben, bei der eine Strahlungsquelle von außerhalb Strahlung in das Behältnis einstrahlt.

Aus der FR 2 815 769 ist eine Elektronenstrahlquelle bekannt, die zwei Elektronenbündel ausstrahlt.

Die DE 198 82 252 T1 beschreibt eine Technik zur Innensterilisation eines Behälters mittels Elektronen. Dabei ist ebenfalls eine Elektronenstrahlquelle vorgesehen, die Strahlung von außerhalb in das Innere des Behältnisses richtet.

Diese genannten Vorrichtungen weisen den Nachteil auf, dass die Strahlung stets von außen durch die Mündung des Behältnisses in dieses eindringt und damit im Inneren des Behältnisses nur schwer hinsichtlich ihrer Strahlrichtung variiert werden kann.

WO-A-2007/095205 beschreibt eine Vorrichtung zum Sterilisieren der Innenseite von Flaschen durch Elektronenbestrahlung. Im oberen Teil des Behandlungskopfes ist eine fokussierende elektrostatische Linse vorgesehen. Der Fokalpunkt befindet sich im Behandlungskopf. Durch diese Anordnung kann die Elektronstrahlung aus der Öffnung nur als eine schmale Kegel austreten.

Aus der EP 0 885 142 B1 ist ein Verfahren zur Sterilisation von Fliesmittelpackungen bekannt. Dabei handelt es sich insbesondere um Verpackungen mit einer offenen Seite. Bei diesem Verfahren sollen hohe Beschleunigungsspannungen vermieden werden, da als unerwünschter Nebeneffekt dieser hohen Beschleunigungsspannungen Röntgenstrahlen erzeugt werden und diese wiederum mit einem Bleimantel abgeschirmt werden müssten. Um gleichermaßen einen Innenreinigungseffekt zu erreichen, wird in der EP 0 885 142 B1 vorgeschlagen, einen Gasstrom zu verwenden, der mit einem Elektronenstrahl in Kontakt steht und auf diese Weise dazu beiträgt, dass die Elektronen an die Innenwandung des Behältnisses gelangen können. Das in der EP 0 885 142 B1 beschriebene Verfahren ist jedoch zur Innenreinigung von Flaschen oder allgemein von Behältnissen mit einem Mündungsdurchmesser ungeeignet, da die in der EP 0 885 142 B1 beschriebene Elektronenstrahlquelle nicht durch diese Mündung hindurchgeführt werden könnte und jedenfalls nicht gemeinsam mit dem in der EP 0 885 142 B1 beschriebenen Luftrohr, welches den Luftstrom im Inneren des Behältnisses erzeugt, in dieses Behältnis eingeführt werden könnte.

Der vorliegenden Erfindung liegt daher die Aufgabe zugrunde, eine effiziente Vorrichtung und ein Verfahren zur Innenreinigung von Behältnissen mit im Vergleich zu dem Behältnisquerschnitt engerem Mündungsquerschnitt zur Verfügung zu stellen.

Dies wird erfindungsgemäß durch eine Vorrichtung nach Anspruch 1, eine Anordnung nach Anspruch 7 und ein Verfahren nach Anspruch 14 erreicht. Vorteilhafte Ausführungsformen und Weiterbildungen sind Gegenstand der Unteransprüche.

Die erfindungsgemäße Vorrichtung zum Sterilisieren von Behältnissen weist einen Behandlungskopf auf, der wiederum ein Austrittsfenster aufweist, durch welches Ladungsträger hindurchtreten können. Weiterhin ist eine Ladungsträgererzeugungsquelle vorgesehen, die Ladungsträger erzeugt und eine Beschleunigungseinrichtung, welche die Ladungsträger in Richtung des Austrittsfensters beschleunigt. Erfindungsgemäß ist der Querschnitt des Behandlungskopfes derart beschaffen, dass der Behandlungskopf durch die Mündung des Behältnis führbar ist und die Beschleunigungseinrichtung beschleunigt die Ladungsträger derart, dass die aus dem Austrittsfenster austretenden Ladungsträger bevorzugt direkt auf eine Innenwandung des Behältnisses richtbar sind.

Im Gegensatz zu dem zitierten Stand der Technik werden damit Beschleunigungsspannungen verwendet, die hoch genug sind, dass der austretende Elektronenstrahl direkt auf die Innenwandung des Behältnisses auftrifft, ohne dass durch Zwischenschaltung eines Gasstroms dieser durch den Elektronenstrahl für eine sterilisierende Wirkung aktiviert wird. Unter einen Austrittsfenster wird eine Einrichtung verstanden, welche zwar den Innenraum der Vorrichtung, d. h. den Bereich zwischen der Ladungsträgererzeugungsquelle und dem Austrittsfenster luftdicht abschließt, durch die jedoch gleichwohl die Ladungsträger insbesondere die Elektronen durchtreten können. Es ist zu beachten, dass die aus dem Austrittsfenster austretenden Elektronen stark gebremst werden, sodass im Gegensatz zu der aus der EP 0 885 142 B1 bekannten Vorrichtung signifikant höhere Energien bzw. Beschleunigungsspannungen verwendet werden.

Zur Erzeugung des Elektronenstrahls dient bevorzugt eine kompakte Elektronenstrahleinheit mit einem Strahlfinger der, wie oben erwähnt, so dimensioniert ist, dass er in die Flasche eintauchen kann um damit eine Elektronenwolke mit möglichst geringer Energie auf die innere Oberfläche der (PET)-Flaschen aufzubringen. Im Falle einer Rundläuferanordnung wäre es möglich, dass Betriebseinheiten für die Vorrichtung, wie beispielsweise Transformatoren oder Netzgeräte für die Elektronenstrahlerzeuger auf einen Karussell mitlaufen, wodurch gleichzeitig die Zuführung von Hochspannung vereinfacht wird. Bei einer bevorzugten Ausführungsform sind das Austrittsfenster und die Beschleunigungseinrichtung innerhalb eines Außengehäuses angeordnet und dieses Außengehäuse ist derart beschaffen, dass es durch die Mündung des Behältnisses führbar ist. Der Behandlungskopf ist damit am unteren Ende dieses Außengehäuses vorgesehen bzw. es ist auch möglich, dass der Behandlungskopf mit dem Außengehäuse einteilig ausgebildet ist. Bevorzugt weist damit das gesamte Außengehäuse mit dem Behandlungskopf einen Querschnitt auf, der durch die Mündung eines Behältnisses einführbar ist. Der Behandlungskopfquerschnitt kann beliebig ausgeformt sein. Besonders bevorzugt weist die Vorrichtung einen kreisförmigen Querschnitt mit einem Durchmesser auf, der unter 40 mm, bevorzugt unter 30 mm und besonders bevorzugt unter 25 mm liegt.

Bevorzugt ist die gesamte Vorrichtung von einer Abschirmungseinrichtung umgeben und insbesondere von einem Bleimantel, um den Austritt von Röntgenstrahlung und damit schädliche Wirkungen auf die Benutzer zu verhindern.

Bei einer weiteren bevorzugten Ausführungsform weist die Vorrichtung ein Innengehäuse auf, innerhalb dessen die Beschleunigungseinrichtung angeordnet ist und das Außengehäuse umgibt dieses Innengehäuse. Der Innenraum dieses Innengehäuses, in dem bevorzugt die Ladungsträgererzeugungsquelle und die Beschleunigungseinrichtung vorgesehen sind, werden besonders bevorzugt mit einem Vakuum beaufschlagt. Besonders bevorzugt wird zwischen dem Außengehäuse und dem Innengehäuse ein sich bis zu dem Behandlungskopf erstreckender Raum gebildet, durch den ein gasförmiges Medium führbar ist. Dieses gasförmige Medium dient insbesondere zum Kühlen des Austrittfensters und zu diesem Zweck wird das gasförmige Medium entlang des Innengehäuses bis zu dem Behandlungskopf und auch vorbei an dem Austrittsfenster geführt. Vorzugsweise wird damit zwischen dem Außengehäuse und dem Innengehäuse ein Raum in Form eines Kanals oder mehrerer Kanäle gebildet, wobei besonders bevorzugt ein unterer Endabschnitt des äußeren Gehäuses so gestaltet ist, dass der Gasstrom auch beispielsweise in radialer Richtung der Vorrichtung und damit auch an dem Austrittsfenster vorbeigeführt wird. Es wird darauf hingewiesen, dass der Strom des gasförmigen Mediums nicht und zumindest nicht unmittelbar an die Innenwandung des Behältnisses gelangt, sondern, wie gesagt, insbesondere dazu dient, um an dem Austrittsfenster vorbeigeführt zu werden. Vorteilhaft ist das gasförmige Medium aus einer Gruppe von gasförmigen Medien ausgewählt, welche Helium, Stickstoff, Argon, Kohlendioxid, Mischungen hieraus oder dergleichen enthält.

Das Austrittsfenster ist aus Titan hergestellt.

Damit ist bei dieser Ausführungsform zumindest ein Teil der Vorrichtung doppelwandig ausgeführt, wobei zwischen dem Außengehäuse und dem Innengehäuse ein Gas oder Luft zur Kühlung des Stahlrohres, d. h. des Innengehäuses und des Fensters ermöglicht wird. Wie erwähnt ist das Innere des Innengehäuses evakuiert und trägt als Austrittsfenster am Ende ein Titanfenster. Das Austrittsfenster weist eine Dicke auf, welche zwischen 5 µm und 20 µm liegt. Damit ist es möglich, für das Austrittfenster eine Fensterfolie beispielsweise aus Titan mit Dicken von 8 µm, 10 µm, 13 µm bzw. 15 µm zu verwenden. Dieses Austrittsfenster bzw. die Fensterfolie wird mit dem inneren Gehäuse, welches ebenfalls aus geeignetem Material (wie z. B. ebenfalls aus Titan) besteht, vakuumdicht verschweißt. Dabei ist es möglich, dass das Austrittsfenster freitragend über die Öffnung des Innengehäuses gespannt ist, es ist jedoch auch möglich, dass eine Stützkonstruktion wie beispielsweise eine Lochplatte vorgesehen ist, welche das Austrittsfenster trägt. Im Fall der Verwendung einer Stützkonstruktion für das Austrittsfenster ist es auch denkbar, dass diese Stützkonstruktion durch eine geeignete Flüssigkeit gekühlt wird, die durch einen oder mehrere Kanäle zwischen dem Innengehäuse und dem Außengehäuse zur Stützkonstruktion hin und wieder abgeführt wird.

Bei einer bevorzugten Ausführungsform weist das Austrittsfenster über seine Fläche hinweg eine gleichmäßige Dicke auf. Es wäre jedoch auch je nach Anwendungsfall denkbar, dass die Dicke variiert, beispielsweise von außen nach innen zunimmt. So ist es möglich, dass die Fensterfolie auch über ihre Fläche hinweg unterschiedliche Dicken aufweist beispielsweise schwankend zwischen 4 und 13 µm oder auch in Form einer Dickenperforation in Linien oder Punkten. Auf diese Weise ist es möglich, unterschiedlich schnelle Elektronen an der Atmosphäre, d. h. der Außenseite des Austrittsfensters zu erhalten und damit ein von einem üblichen Streufeld abweichendes Streufeld.

Wie oben erwähnt, können als Kühlgas unterschiedliche Gase eingesetzt werden. Auf diese Weise kann eine verbesserte Wärmeleitung der Gase erreicht werden, insbesondere mit Helium aber es wäre auch möglich, die Bedingungen für die Elektronen an der Atmosphäre d. h. nach dem Austritt aus dem Austrittsfenster zu beeinflussen. So führt beispielsweise die Verwendung von Helium als Atmosphäre zu größeren Reichweiten der Elektronen, da Helium eine geringere Dichte aufweist als Luft. Weiterhin kann durch die Zuführung der Gase auch eine Inertisierung des Behandlungsraums d. h. des Innenraums des Behältnisses, erreicht werden, was wiederum zu einer Verminderung der Ozonentstehung führt.

Als Strahlerzeuger kann sowohl eine offene als auch eine geschlossene Elektronenstrahleinheit, die beispielsweise aus Röntgenröhren oder ähnlichem bekannt ist, verwendet werden. Die Elektronenquelle selbst kann aus punktförmige oder flächige Elektronenquelle ausgeführt sein, an die das Innengehäuse bzw. der Strahlfinger direkt angebracht ist.

Die erfindungsgemäße Vorrichtung wird im Folgenden auch als Strahlfinger bezeichnet.

Die Vorrichtung weist eine Ablenkeinheit zum Ablenken der Ladungsträger auf. Besonders bevorzugt ist diese Ablenkeinrichtung zwischen der Ladungsträgererzeugungsquelle und dem Austrittsfenster angeordnet. Damit kann eine elektromagnetische Ablenkung der Elektronen vor dem Austrittfenster stattfinden, um den Elektronen zusätzlich eine Vorzugsrichtung in Richtung der Flaschenwand zu geben. Auf diese Weise ist es möglich, die Strahlungsdosis gleichmäßiger über die Innenoberfläche des Behältnisses zu verteilen. Auch ist es auf diese Weise möglich, den Elektronenstrahl gleichmäßig über die gesamte Austrittsfläche des Austrittsfensters zu führen um auf diese Weise eine gleichmäßigere Belastung des Austrittfensters in seiner Gesamtheit zu erreichen bzw. um damit sog. Hotspots auf dem Fenster zu verhindern. Es ist jedoch auch denkbar, dass die Ablenkeinheit in Richtung des Elektronenstrahls nach dem Austrittsfenster angeordnet ist.

Bevorzugt weist das Innengehäuse im Bereich des Behandlungkopfes einen vergrößerten Innendurchmesser auf, um diese erwähnte Ablenkung der Ladungsträger zu vereinfachen.

Weiterhin ist besonders bevorzugt der Behandlungskopf gegenüber den Behältnissen in einer Längsrichtung des Behältnisses bewegbar. Auf diese Weise kann in einem Arbeitsgang ein größerer flächiger Bereich der Innenwandung des Behältnisses sterilisiert werden.

Bei einer weiteren vorteilhaften Ausführungsform ist der Behandlungskopf gegenüber dem Behältnis drehbar. Dies ist insbesondere dann vorteilhaft, wenn die Ladungsträger auch in einer bevorzugten radialen Richtung bezüglich des Behandlungskopfes austreten. Bei einer weiteren vorteilhaften Ausführungsform ist der Behandlungskopf bezüglich dem Behältnis in einer radialen Richtung des Behältnisses bewegbar. So ist es beispielsweise möglich, dass während des Sterilisationsvorgangs der Behandlungskopf selbst bzw. die Vorrichtung um eine vorgegebene Schwenkachse geschwenkt wird oder auch, dass das Behältnis selbst gegenüber dem Behandlungskopf geschwenkt wird. Besonders bevorzugt ist dabei eine Schwenkachse in einem Bereich der Mündung des Behältnisses bzw. geringfügig oberhalb der Mündung des Behältnisses vorgesehen. Auf diese Weise kann der Behandlungskopf in die Nähe der Innenwandung des Behältnisses gebracht werden.

Die vorliegende Vorrichtung betrifft auch eine Anordnung nach Anspruch 7.

Vorzugsweise weist die Anordnung eine Einrichtung zum Befüllen von Behältnissen auf und die erfindungsgemäße Vorrichtung ist stromaufwärts bezüglich dieser Einrichtung angeordnet.

Weiterhin kann die Anordnung eine Verschiebeeinrichtung aufweisen, welche die Behältnisse in der Längsrichtung der Behältnisse gegenüber der Vorrichtung verschiebt. Grundsätzlich wäre es auch möglich, die Höhenlage der Behältnisse beizubehalten und lediglich die Vorrichtung in der Längsrichtung der Behältnisse zu verschieben. Da jedoch die Vorrichtung bzw. der Strahlfinger relativ aufwendig gestaltet und üblicherweise auch mit Hochspannungen beaufschlagt sind, bietet es sich eher an, diese stationär zu halten und vielmehr die Behältnisse gegenüber der Vorrichtung zu verschieben.

Weiterhin ist vorteilhaft die Vorrichtung zwischen einer Expansionseinrichtung für die Behältnisse und einer Einrichtung zum Befüllen der Behältnisse angeordnet. Damit wird eine Sterilisation dieser Anordnung nach dem Expandieren der Behältnisse bzw. den Blasen der Behältnisse durchgeführt.

Die Anordnung weist eine Vielzahl von Vorrichtungen der oben beschriebenen Art auf, wobei diese einzelnen Vorrichtungen entlang einer Kreisbahn angeordnet sind.

Bei einer weiteren vorteilhaften Ausführungsform weist die Anordnung eine weitere Sterilisationsvorrichtung zum Sterilisieren einer Außenwandung der Behältnisse auf. Auf diese Weise ist es möglich, nicht nur eine Innensterilisation sondern auch eine Außensterilisation der Behältnisse durchzuführen.

Dabei ist besonders bevorzugt die weitere Sterilisationsvorrichtung in der Transportrichtung der Behältnisse stromaufwärts der oben genannten Vorrichtungen angeordnet. Dies bedeutet, dass bevorzugt zunächst eine Außensterislisation der Behältnisse und anschließend Innensterilisation vorgenommen wird. Bevorzugt handelt es sich auch bei der weiteren Sterilisationsvorrichtung um eine Vorrichtung, welche die Behältnisse mit Ladungsträgern bestrahlt. Daneben oder zusätzlich wären jedoch auch andere Prinzipien der Sterilisation anwendbar.

Bevorzugt ist die weitere Sterilisationsvorrichtung stationär angeordnet. Dies bedeutet, dass die Behältnisse an dieser stationär angeordneten Sterilisationsvorrichtung vorbeigeführt werden. Bevorzugt werden durch die weitere Sterislisationsvorrichtung insbesondere die Umfangswandung und gegegebenenfalls auch der Halsbereich der Behältnisse sterilisiert.

Bei einer weiteren vorteilhaften Ausführungsform ist die weitere Sterilisationsvorrichtung in einem Sterilraum angeordnet.

Bei einer weiteren bevorzugten Ausführungsform weist die Anordnung ein Transportkarussell auf, welches die Behältnisse an der weiteren Sterilisationsvrrichtung vorbeiführt. Damit werden die Behältnisse auf einer kreisförmigen Bahn geführt. Genauer gesagt, ist bevorzugt eine Vielzahl von Transportkarussels vorgesehen, welche die Behältnisse einander übergeben. Bevorzugt weist dieses Transportkarussell Dreheinrichtungen auf, welche die Behältnisse um ihre Längsachse drehen. Durch das Vorsehen dieser Dreheinrichtungen ist es möglich, einen größeren Umfangsbereich der Behältnisse zu sterilisieren.

Die vorliegende Erfindung betrifft auch ein Verfahren nach Anspruch 14.

Die Ladungsträger werden vor Erreichen des Austrittfensters in einer radialen Richtung des Behältnisses bzw. der Vorrichtung abgelenkt. Zu dieser Ablenkung können Spulen oder dergleichen eingesetzt werden.

Das Austrittsfenster wird gekühlt und als Kühlmedium zum Kühlen des Austrittsfensters wird ein gasförmiges Medium verwendet.

Weitere Vorteile und Ausführungsformen ergeben sich aus den beigefügten Zeichnungen:
Darin zeigen:
   - Fig. 1a: eine teilweise Ansicht einer erfindungsgemäßen Vorrichtung zum sterilisieren von Behältnissen;
   - Fig. 1b: ein Außengehäuse für die Vorrichtung aus Fig. 1a;
   - Fig. 1c: ein Innengehäuse für die Vorrichtung aus Fig. 1a;
   - Fig. 1d: einen Stützkörper mit Austrittsfenster für die Vorrichtung aus Fig. 1a;
   - Fig. 1e: eine Draufsicht auf den Stützfenster aus Fig. 1d;
   - Fig. 1f: eine Draufsicht auf das Austrittsfenster aus Fig. 1a;
   - Fig. 1g: eine weitere Ausführungsform eines Stützfensters;
   - Fig. 2: eine weitere Ausführungsform einer erfindungsgemäßen Vorrichtung;
   - Fig. 3: eine weitere Ausführungsform einer erfindungsgemäßen Vorrichtung;
   - Fig. 4: eine Anordnung zum Behandeln von Behältnissen;
   - Fig. 5: ein weiteres Beispiel für eine Anordnung zum Behandeln von Behältnissen;
   - Fig. 6: eine Detailansicht einer Anordnung zum Behandeln von Behältnissen;
   - Fig. 7: ausschnittsweise schematische Seitenansicht einer Anordnung zum Behandeln von Behältnissen und
   - Fig. 8: eine weitere Ausführungsform einer erfindungsgemäßen Vorrichtung.
   - Fig. 9: eine perspektivische Ansicht einer erfindungsgemäßen Anordnung;
   - Fig. 10: eine Draufsicht auf die Anordnung aus Fig. 9;
   - Fig. 11: eine Detailansicht der Vorrichtung aus Fig. 9;
   - Fig. 12: eine weitere Detailansicht der Vorrichtung aus Fig. 9;
   - Fig. 13: eine perspektivische Ansicht der Vorrichtung aus Fig. 9;
   - Fig. 14: die Vorrichtung aus Fig. 13 mit einer Einhausung;
   - Fig. 15: eine Draufsicht auf eine erfindungsgemäße Anordnung in einer weiteren Ausführungsform; und
   - Fig. 16: eine perspektivische Ansicht der Vorrichtung aus Fig. 15.

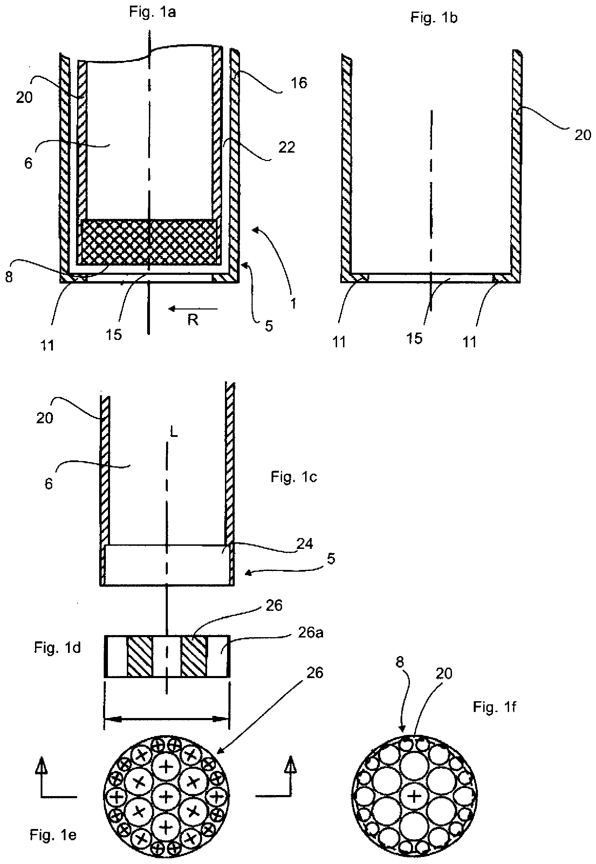

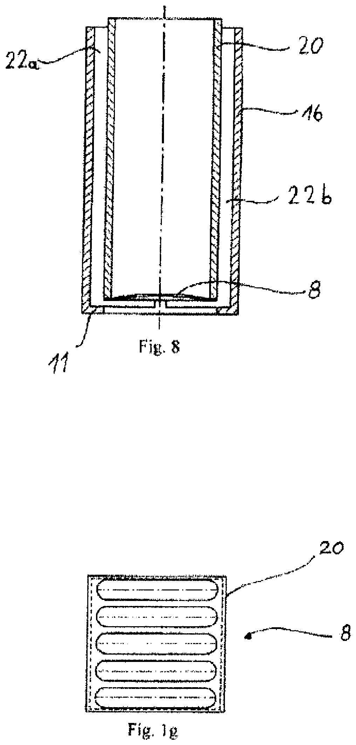

Fig. 1a zeigt eine ausschnittsweise Darstellung einer erfindungsgemäßen Vorrichtung zum Sterilisieren von Behältnissen. Diese Vorrichtung weist an ihrem unteren Ende einen Behandlungskopf 5 auf, an dem ein Austrittsfenster 8 vorgesehen ist, über welches ein Elektronenstrahl austreten kann. Dabei werden, wie aus dem Stand der Technik üblich, zunächst beispielsweise mit Hilfe einer Wolframkathode die Elektronen erzeugt. Diese Elektronen werden anschließend über eine (nicht im Detail gezeigte) Beschleunigungseinrichtung 6 beschleunigt. Als Elektronenerzeugungsquelle können dabei punktförmige oder auch flächige Elektronenquellen verwendet werden.

Die Vorrichtung 1 zum Sterilisieren von Behältnissen weist ein Außengehäuse 16 und ein Innengehäuse 20 auf, ist also doppelwandig ausgeführt. Zwischen dem Außengehäuse 16 und dem Innengehäuse 20 ist ein Spalt 22 gebildet, entlang dessen Luft, ein anderes gasförmiges Medium oder auch ein Flüssiges Medium geführt werden kann. Dabei kann dieser Luftspalt 22 in Umfangsrichtung umlaufend ausgebildet sein, es ist jedoch auch möglich, dass eine Vielzahl von Kanälen 22 vorgesehen ist. Prinzipiell ist es dabei möglich, dass der Gasstrahl während des Betriebs der Vorrichtung 1 in radialer Richtung R an dem Austrittsfenster 8 vorbeigeführt wird, es ist jedoch auch möglich, dass der Gasstrom in dem Zeitraum an dem Austrittsfenster 8 vorbeigeführt wird, in dem die Vorrichtung 1 nicht aktiv ist bzw. die Strahlungsquelle nicht aktiv ist. Auf diese Weise kann verhindert werden, dass der austretende Elektronenstrahl durch den Luftstrom beeinflusst wird. Es wird dabei darauf hingewiesen, dass der Gasstrom ausschließlich zum Kühlen des Austrittfensters dient und nicht zum Führen des Elektronenstrahls.

Grundsätzlich ist der Strahlstrom im Falle einer optimal gewählten Beschleunigungsspannung der maßgebenende Faktor, um in möglichst kurzer Zeit die entsprechend notwendige Dosis im Behältnis zu erzeugen. Dieser Strahlstrom führt jedoch in dem Austrittsfenster 8 zu Verlusten, die je nach Ausführung dieses Austrittsfensters 8 früher oder später auch die maximale Strahlleistung der Elektronenstrahleinheit bzw. der Vorrichtung 1 begrenzen. Mit der beschriebenen Luft-, Gas- oder Flüssigkeitskühlung kann jedoch auch die notwendige Kühlung des Austrittsfensters gewährleistet werden. Mit anderen Worten soll, um den maximal möglichen Strahlstrom zu erreichen d. h. den maximal möglichen Durchsatz, die Anzahl der Strahleinheiten minimiert bzw. die Taktzeit erhöht werden. Auch wäre es möglich, die Streugeometrie an der Atmosphäre d. h. außerhalb des Austrittsfensters 8 zu verbessern.

Die Elektronen werden auf Energie in einen Bereich von 100 keV - 200 keV vorzugsweise zwischen 120 keV und 180 keV und vorzugsweise 130 keV und 170 keV beschleunigt.

Fig. 1b zeigt ein Außengehäuse 20 für eine erfindungsgemäße Vorrichtung 1. Man erkennt, dass am unteren Ende des Außengehäuses 20 in radialer Richtung nach innen ragende Wände 11 vorgesehen sind. Diese Wände 11 dienen zur Luft bzw. Gasführung d. h. sie bewirken, dass Gas wenigstens auch abschnittsweise Austrittsfenster 8 vorbeigeführt werden. Durch diese Wände 11 wird weiterhin eine Öffnung 15 gebildet, durch welche auch der entstehende Elektronenstrahl nach außen treten kann.

Es wäre jedoch auch möglich, auf einer Seite bezüglich des Innengehäuses 20 Gas zuzuführen und dieses auf der anderen Seite des Innengehäuses 20 wieder abzuführen.

Fig. 1c zeigt ein Innengehäuse 20. Dieses Innengehäuse 20 weist an seinem unteren Ende bzw. an dem Behandlungskopf 5 eine Ausnehmung 24 auf, um das Austrittsfenster 8 bzw. auch einen Stützkörper 26 des Austrittsfensters 8 aufzunehmen.

Fig. 1d zeigt einen Stützkörper 26, der zum Stützen des eigentlichen in Fig. 1f gezeigten Austrittsfensters 8 dient. Bei dieser Ausführungsform bildet dieser Stützkörper 26 eine Vielzahl von Kanälen 26a, durch welche hindurch der Elektronenstrahl treten kann. Zusätzlich kann der Stützkörper 26 Kanäle aufweisen, durch die hindurch eine Kühlfüssigkeit oder ein Kühlgas geführt werden kann.

Das in Fig. 1f gezeigte eigentliche Austrittsfenster 8 ist beispielsweise auf den Stützkörper 26 angeschweißt.

Für das Innengehäuse 20 bzw. die gesamte Vorrichtung 1 sind unterschiedliche Ausführungsformen denkbar. So ist es möglich, die Vorrichtung beispielsweise aus Quarzglas auszuführen, d. h. insbesondere das Innenrohr 20 des Quarzglases auszuführen. Dabei kann beispielsweise eine dünne Quarzglasfolie als Austrittsfenster 8 eingeschmolzen sein. Aus dem Stand der Technik sind bereits derartige Folien mit einer Dicke von 20 µm bekannt. Die Dichte derartiger Fenster liegt in einem Bereich von 2,2 g/cm³bzw. entsprechend 44 g/cm². Dies entspricht der Flächendichte einer Titanfolie mit einer Dicke von 10 µm.

Derartige Quarzglasfolien erlauben Betriebtemperaturen von bis 1000° C d. h. zur Kühlung ist auch ein hoher Temperaturgradient möglich. Ein weiterer Vorteil derartiger Glasfolien aus Quarzglas ist, das Quarzglasfolien in dieser Dicke elastisch sind.

Eine weitere Möglichkeit bestünde darin, die Vorrichtung 1 bzw. das Innengehäuse 20 aus Quarzglas auszuführen und das Austrittsfenster 8 aus Diamant. Auch hier sind bereits Folien mit einer Dicke von 10 µm bekannt. Derartige Folien weisen eine Dichte im Bereich von 3,5 g/cm³bzw. 35 g/cm² auf. Der Vorteil derartiger Folien aus Diamant sind eine weiter verbesserte Wärmeleitfähigkeit, die beispielsweise die Wärmeleitfähigkeit von Kupfer um den Faktor 5 übertrifft.

Weiterhin wäre es auch möglich, die Vorrichtung 1 d. h. insbesondere das Innenrohr bzw. Innengehäuse 20 aus Metall (z. B. Titan) auszuführen und das Austrittsfenster 8, wie oben erwähnt, aus Diamant mit den oben bezeichneten Eigenschaften. Dabei wäre es möglich, dass das Austrittsfenster 8 eingelötet oder verschmolzen ist. Falls Glasrohre verwendet werden, sollten diese vorteilhaft metallisiert werden, da über die vergleichsweise lange Strecke Aufladungen auftreten können.

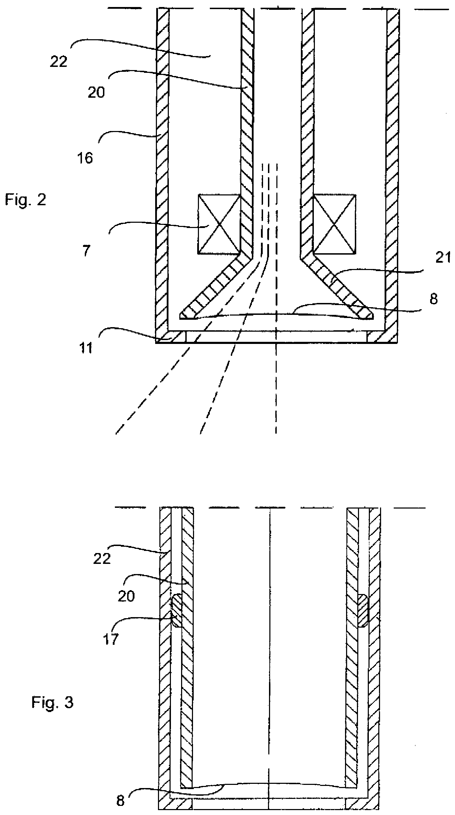

Fig. 2 zeigt eine weitere Ausführungsform einer erfindungsgemäßen Vorrichtung 1. In diesem Falle weist das Innengehäuse 20 bzw. Innenrohr im oberen Bereich einen relativ kleinen Innenquerschnitt auf, der sich im Bereich des Behandlungskopfes 5 erweitert. Auf diese Weise wird ein relativ großer Raum 22 zwischen dem Innengehäuse 20 und dem Außengehäuse 16 gebildet. Im Bereich des Behandlungskopfes 5 weist das Innengehäuse einen sich kegelförmig erweiterten Endabschnitt 21 auf. Durch diesen Endabschnitt 21 ist es möglich, die Elektronenstrahlen unter relativ hohem Winkel zu lenken. Zur Ablenkung des Elektronenstrahls können beispielsweise Ablenkspulen 7 vorgesehen sein. Da neben dem in Fig. 2 gezeigten Spulenpaar noch ein weiteres senkrecht stehendes Spulenpaar angeordnet ist, können die Elektronenstrahlen im Prinzip in alle Raumrichtungen ausgesandt werden. Bei der in Fig. 2 gezeigten Ausführungsform ist lediglich ein Austrittsfenster 8 in Form einer Titanfolie vorgesehen. Es könnte jedoch auch hier ein Stützkörper 26 wie in den Figuren 1a bzw. 1e vorgesehen sein.

Anstelle der in Fig. 2 gezeigten kegelförmigen Erweiterung könnte jedoch der untere Bereich 21 auch beispielsweise halbkugelförmig ausgebildet sein. Weiterhin wäre es jedoch auch möglich, dass die Ablenkspulen außerhalb des äußeren Gehäuses 16 angeordnet sind. Daneben wäre es auch möglich, die Ablenkspulen erst unterhalb der Vorrichtung 1 anzuordnen. Bei der in Fig. 2 gezeigten Ausführungsform ist auch das Innengehäuse 20 aus Titan gebildet.

Fig. 3 zeigt eine weitere Ausführungsform für eine erfindungsgemäße Vorrichtung. Bei dieser Vorrichtung ist ein relativ dünner Luftspalt 22 zwischen dem Innengehäuse 20 und dem Außengehäuse 16 vorgesehen. Um Kontakte zwischen dem Innengehäuse 20 und dem Außengehäuse 16 zu vermeiden, ist es möglich, zwischen den beiden Gehäusen 16, 20 Abstandskörper 17 anzuordnen, wobei die Abstandskörper 17 vorteilhaft elektrisch isoliert sind. Wie bei der in Fig. 2 gezeigten Ausführungsform könnten auch bei der in Fig. 3 gezeigten Ausführungsform Ablenkspulen 7 zur Ablenkung des Elektronenstrahls vorgesehen sein.

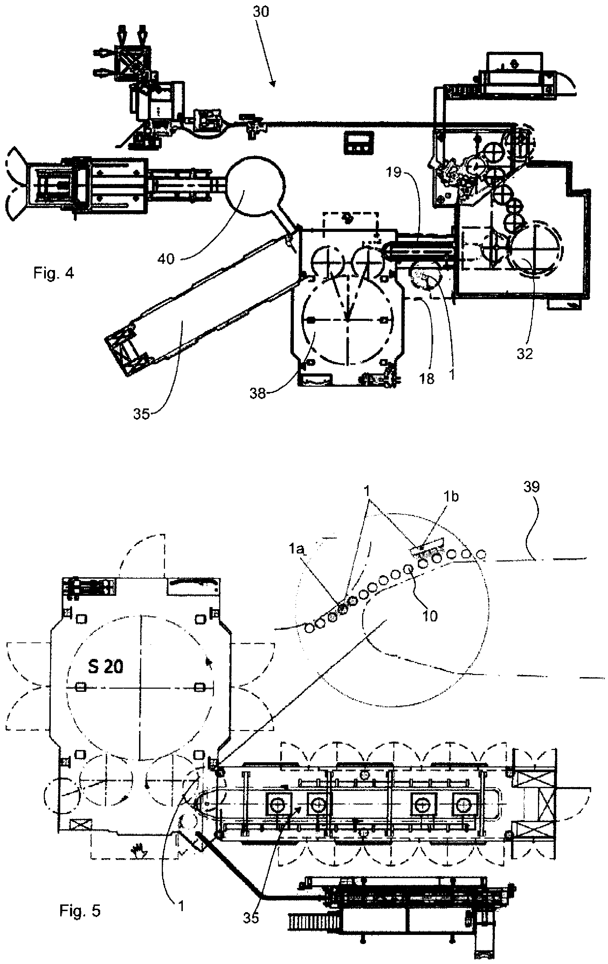

Fig. 4 zeigt eine erfindungsgemäße Anordnung zum Behandeln von Behältnissen. Diese Anordnung weist Sortiereinrichtung 40 für Vorformlinge, vorzugsweise eine als rotierende Scheibe ausgebildete Sortiereinrichtung 40 sowie eine Heizstrecke bzw. einen Ofen 35 auf, in dem Vorformlinge erwärmt werden. An diese Aufwärmstrecke schließt sich eine Blasvorrichtung 38 an, welche die Vorformlinge zu Behältnissen bläst. Im Anschluss auf diese Blasvorrichtung befindet sich eine bzw. eine Vielzahl von erfindungsgemäßen Vorrichtungen 1 zum Sterilisieren der bereits geblasenen Behältnisse. Dabei bezieht sich das Bezugszeichen 18 auf eine Abschirmeinrichtung, wie einen Bleimantel, der die gesamte Vorrichtung 1 umgibt, um den Austritt von Röntgenstrahlen zu verhindern.

Das Bezugszeichen 19 bezieht sich auf ein Transportelement, welches die Behältnisse von der Blasvorrichtung 38 zu einer Fülleinrichtung 32 transportiert. Damit sind in der Bewegungsrichtung der Behältnisse zunächst eine Blasvorrichtung 38 anschließend eine Fülleinrichtung 32 und möglicherweise anschließend noch eine Etikettiermaschine vorgesehen. Es wäre jedoch auch möglich, dass auf die Blasvorrichtung 38 zunächst eine Etikettiermaschine und anschließend eine Fülleinrichtung 32 folgt. Bei der erfindungsgemäßen Anordnung ist die Vorrichtung 1 jeweils unmittelbar nach der Blasvorrichtung 38 angeordnet.

Es wäre weiterhin möglich, neben der erfindungsgemäßen Vorrichtung 1 noch andere ähnlich geartete Sterilisationsvorrichtungen vorzusehen, welche zu der Außenreinigung d. h. der Sterilisation der Außenwände der Behältnisse dienen. Die einzelnen Behältnisse können zur Innensterilisation auf einem Rundläufer geführt werden und dann jeweils die erfindungsgemäßen Vorrichtungen in die Behältnisse eingetaucht werden. Dabei ist es, wie oben erwähnt, vorteilhaft, die einzelnen Vorrichtungen bzw. Strahlfinger auf einem konstanten Höhenniveau zu halten und die Behältnisse diesen gegenüber zu bewegen.

Mit anderen Worten werden die Strahlfinger 1 für die Innensterilisation in die Behältnisse eingetaucht. besonders bevorzugt werden die einzelnen Behältnisse rotierend an einzelnen erfindungsgemäßen Vorrichtungen vorbeigefahren. Es ist jedoch zusätzlich auch möglich, die einzelnen Behältnisse an einem Vorhang mit Elektronenstrahlen vorbeizufahren, um auf diese Weise eine Außensterilisation zu bewirken.

Fig. 5 zeigt eine Detailansicht für eine weitere erfindungsgemäße Anordnung einer Sterilisationsvorrichtung.

Grundsätzlich ist es möglich, dass die Behältnisse über eine Kette transportiert bzw. über eine Kette in einen Behandlungsraum geführt werden und dort Strahlfinger über eine Hubbewegung in die Flaschen eintauchen. Es wäre jedoch auch möglich, die Behältnisse über einen Stern im Neckhandling an eine Sterilisationseinrichtung zu übergeben und dort über eine Hubbewegung über den Strahlfinger zu bewegen.

Bei der in Fig. 5 gezeigten Ausführungsform erfolgt zunächst durch eine Bestrahlungsvorrichtung 1b eine Außenbestrahlung der Behältnisse, bevorzugt während einer Rotation derselben. Dabei werden zur Bestrahlung jeweils auf ca. 150 keV beschleunigte Elektronenstrahlen verwendet. Anschließend wird durch die Vorrichtung 1a in der oben beschriebenen Weise eine Innenbestrahlung der Behältnisse durchgeführt.

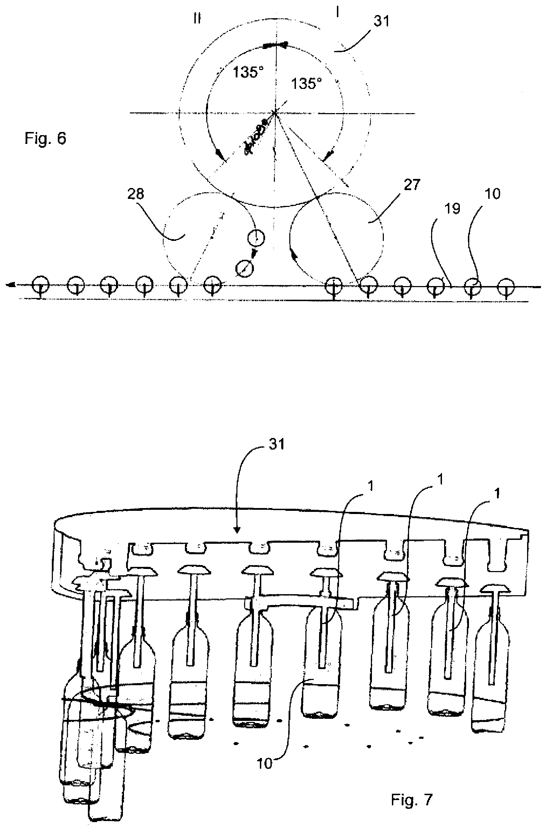

Fig. 6 zeigt eine schematische Veranschaulichung eines erfindungsgemäßen Verfahrens für die Innensterilisation von Behältnissen 10. Die Behältnisse 10 werden über ein Transportband 19 in Richtung des Pfeils P antransportiert und über einen Einlaufstern 27 zu einer Sterilisationsanordnung gebracht. In einem ersten Sektor I erfolgt zunächst über einen Ausfahrwinkel von 135° ein Herabsenken der Strahlfinger in das Innere der Behältnisse 10 bzw. ein Anheben der Behältnisse 10. In einem weiteren Sektor II werden die Strahlfinger wieder zurückgefahren bzw. die Behältnisse 10 werden abgesenkt. Während dieses Ausfahrens des Strahlfingers aus den Behältnissen 10 wird die Vorrichtung 1 aktiviert und auf diese Weise eine Innensterilisation der Behältnisse 10 vorgenommen.

Anschließend werden die Behältnisse über einen Auslaufstern 28 abtransportiert.

Fig. 7 zeigt eine schematische Darstellung des Umlaufrades 31. Dieses Umlaufrad 31 weist eine Vielzahl von erfindungsgemäßen Vorrichtungen 1 auf, die jeweils auf der gleichen Höhe angeordnet sind, man erkennt jedoch, dass die einzelnen Behältnisse 10 jeweils bis zu einer Maximalposition angehoben werden und auf diese Weise die Vorrichtung 1 bzw. der Strahlfinger in Abhängigkeit von der Drehstellung unterschiedlich tief in die Behältnisse 10 eingetaucht werden. Während dieses gesamten Vorgangs sind die erfindungsgemäßen Vorrichtungen 1 aktiviert und auf diese Weise wird die Innenwandung der Behältnisse 10 über die gesamte Höhe der Behältnisse 1 sterilisiert. Die Länge der Strahlfinger wird dabei so gewählt, dass auch eine effektive Sterilisation der Böden der Behältnisse 10 möglich ist.

Fig. 8 zeigt eine weitere Ausführungsform für eine erfindungsgemäße Vorrichtung. Bei dieser Vorrichtung ist der Bereich zwischen dem Innengehäuse 20 und dem Außengehäuse 16 derart gestaltet, dass sich zwei Gaskanäle 22a und 22b bilden. Der Gasstrom wird durch einen der beiden Kanäle 22a zugeführt und streift seitlich gleichmäßig über das Austrittsfenster 8. Durch den anderen Kanal 22b kann der Gasstrom wieder abgeführt werden.

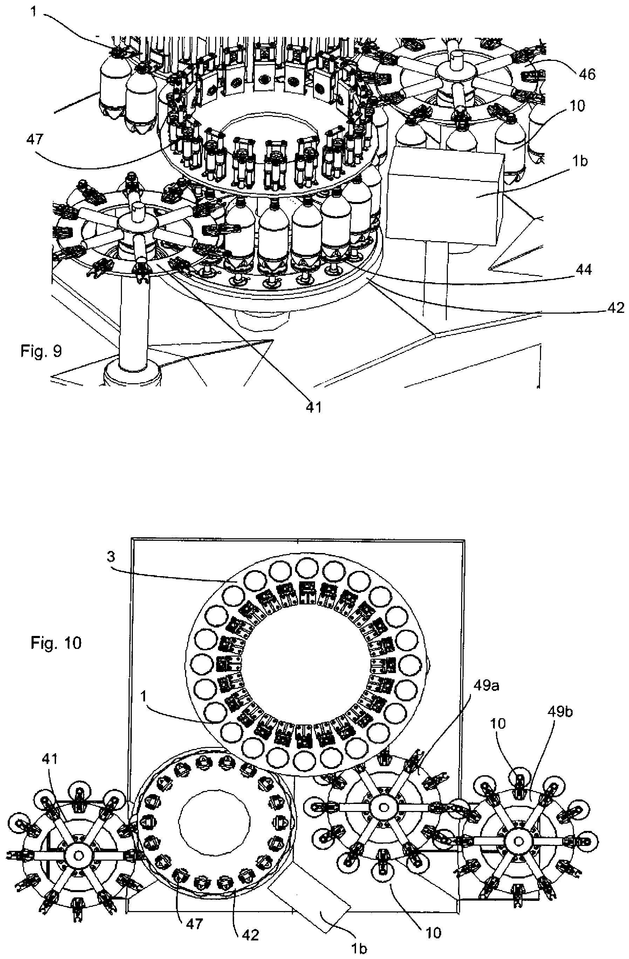

Fig. 9 zeigt eine perspektivische Ansicht einer erfindungsgemäßen Anordnung. Stromaufwärts bezüglich einer Vielzahl von erfindungsgemäßen Vorrichtungen 1 ist ein weiteres Transportkarussell 42 vorgesehen, auf dem die Behältnisse transportiert werden. Dabei werden die Behältnisse an einer weiteren Sterilisationsvorrichtung 1b für Behältnisse vorbeigeführt und von dieser weiteren Sterilisationsvorrichtung 1b an ihrer Außenwandung sterilisiert. Anschließend wird, wie oben beschrieben, der Innenraum der Behältnisse 10 sterilisiert.

Das Bezugszeichen 44 bezieht sich auf Dreheinrichtungen wie Drehteller, mittels denen die Behältnisse 10 um ihre Längsrichtung (bzw. Längsachse) gedreht werden. Durch diese Drehung kann ein größerer Bereich des Aussenumfangs der Behältnisse 10 sterilisiert werden. Auch wäre es möglich, in der Transportrichtung der Behältnisse 10 mehrere Sterilisationsvorrichtungen 1b hintereinander vorzusehen.

Fig. 10 zeigt eine Draufsicht auf die Anordnung aus Fig. 9. Stromabwärts bezüglich eines Transportrades 3, auf dem eine Vielzahl von Vorrichtungen 1 zur Innensterilisation vorgesehen ist, sind zwei weitere Transporträder 49a, 49b vorgesehen, welche die Behältnisse aus der Anordnung heraus transportieren.

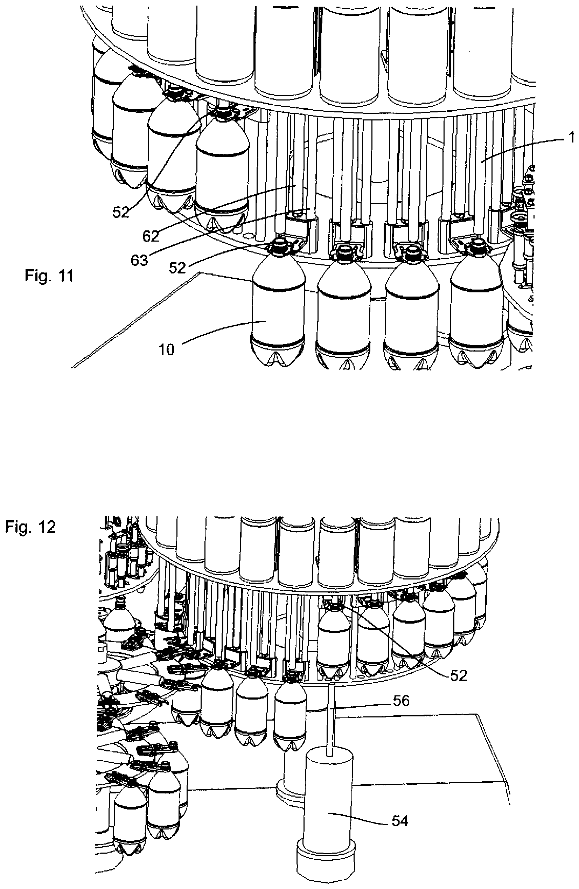

Fig. 11 zeigt eine detaillierte Ansicht einer erfindungsgemäßen Vorrichtung. Man erkennt hier eine Vielzahl von Trägern 52, an denen jeweils die Behältnisse 10 angeordnet sind. Diese Träger 52 sind wiederum an Führungsstangen 62 angeordnet und auf diese Weise können die Behältnisse 10 in Ihrer Längsrichtung bewegt werden. Die Vorrichtungen 1, welche stationär angeordnet sind, dringen bei einem Anheben der Behältnisse 10 weiter in diese ein. Die Bezugszeichen 63 beziehen sich auf Führungsstangen, welche eine Bewegung der Träger 52 führen. Diese Führungsstangen 63 sind vorteilhaft stationär angeordnet.

Fig. 12 zeigt ein weiteres Detail einer erfindungsgemäßen Anordnung. Man erkennt hier eine weitere Sterilisationsvorrichtung 54, welche unterhalb der Behältnisse angeordnet ist und eine Sterilisation des Bodens der Behältnisse 10 bewirkt. Dabei weist auch diese Sterilisationsvorrichtung 54 einen stangenartigen Körper 56 auf, aus dessen Ende der Ladungsträgerstrahl austreten kann. Nach einer Bestrahlung der Behältnisse mit dieser Sterilisationsvorrichtung 54 werden die Behältnisse 10 wieder in ihrer Längsrichtung abgesenkt.

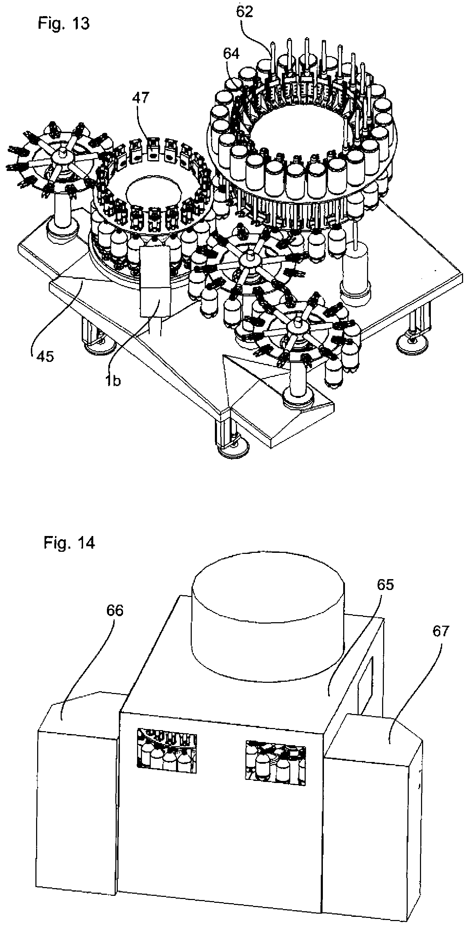

Fig. 13 zeigt eine perspektivische Darstellung einer erfindungsgemäßen Anordnung. Man erkennt hier, dass die oben beschriebene Bodensterilisation nach der Sterilisation des Innenraums durchgeführt wird. Das Bezugszeichen 45 bezieht sich auf einen Träger, an dem die gesamte Anordnung angeordnet ist.

Fig. 14 zeigt eine weitere Darstellung der erfindungsgemäßen Anordnung. Man erkennt, dass hier die einzelnen Transport- und Sterilisationsvorrichtungen in einem Gehäuse bzw. drei Gehäuseteilen 65, 66, 67 angeordnet sind. Auf diese Weise ist es möglich, die gesamte Vorrichtung innerhalb eines sterilen Raums zu halten. Auch können durch die Gehäuseteile 65 - 67 störende Strahlungen abgeschirmt werden.

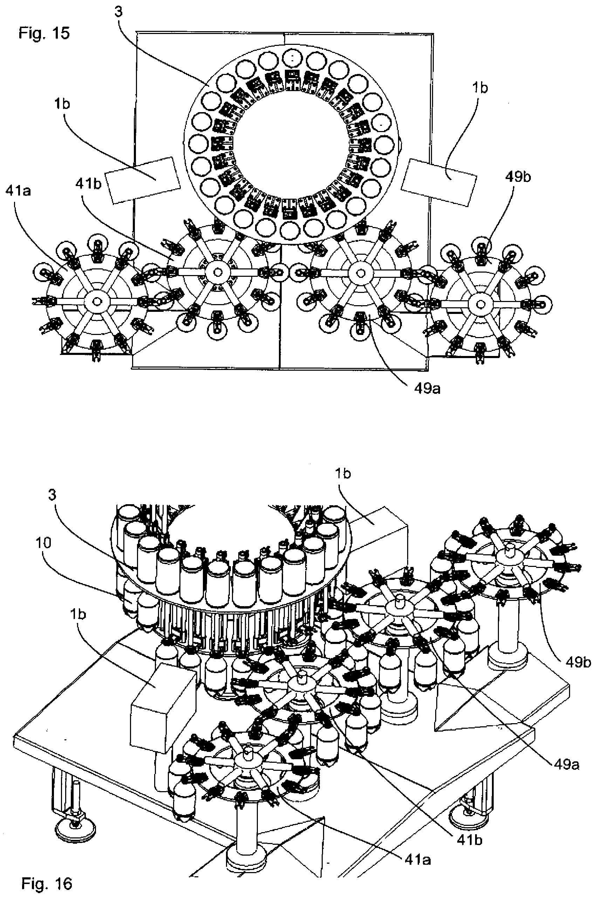

Fig. 15 zeigt eine weitere Ausführungsform einer erfindungsgemäßen Anordnung. Man erkennt hier, dass zwei Sterilisationsvorrichtungen 1b vorgesehen sind, welche die Außensterilisation der Behältnisse übernehmen. Im Gegensatz zu den oben gezeigten Ausführungsformen sind hier die beiden Sterilisationsvorrichtungen 1b nicht stromaufwärts bezüglich der Transporteinrichtung 3, auf der die Innensterilisation der Behältnisse stattfindet, angeordnet, sondern um diese Transporteinrichtung 3 herum.

Genauer findet bei der in Fig. 15 gezeigten Ausfürhungsform die Sterilisation der Aussenwandung sowohl (unmittelbar) vor der Sterilisation der Innenwandung als auch (unmittelbar) danach statt. Die Bezugszeichen 41a und 41b beziehen sich auf Transportsterne, welche die Behältnisse 10 der Transporteinrichtung 3 zuführen und die Bezugszeichen 49a und 49b auf Transportsterne, welche die Behältnisse 10 wieder abtransportieren.

Fig. 16 zeigt eine perspektivische Darstellung der in Fig. 15 gezeigten Vorrichtung. Man erkennt, dass zunächst eine Sterilisation der Außenfläche der Behältnisse durch die links dargestellte Sterilisationsvorrichtung 1b vorgenommen wird, wobei die Behältniss bevorzugt in diesem Bereich um ca. 180° gedreht werden. Anschließend werden die Behältnisse angehoben, um eine Innenraumsterilisation durchzuführen. Danach werden die Behältnisse wieder abgesenkt und es wird abermals der Außenumfang mit der rechten Sterilisationsvorrichtung sterilisiert, wobei hierzu bevorzugt die Behältnisse 10 um weitere 180° gedreht werden. Auch wäre es möglich, die Behältnisse 10 auch während der Innensterilisation zu drehen.

Vorzugsweise sind daher entsprechende Dreheinrichtungen derart aufeinander abgestimmt, dass die beiden Sterilisationsvorrichtungen 1b eine vollständige Außensterilisation der Behältnisse 10 vornehmen können. Damit wird hier nur ein Karussell für die Außen/Innensterilisation verwendet. Durch das Vorsehen zweier Sterilisationsvorrichtungen 1b kann die für die Außensterilisation nötige Drehgeschwindigkeit der Behältnisse 10 reduziert werden. Die in den Fig. 15 und 16 gezeigte Anordnung erlaub damit eine höhere Platzeinsparung.

## Patentansprüche

1. Vorrichtung (1) zum Sterilisieren von Flaschen (10), welche eine Mündung aufweisen,
mit einem Behandlungskopf (5), der ein Austrittsfenster (8) aufweist, durch welches Ladungsträger hindurchtreten können, mit einer Ladungsträgererzeugungsquelle, die
Ladungsträger erzeugt und mit einer Beschleunigungseinrichtung (6) welche die Ladungsträger in Richtung des Austrittsfensters (8) beschleunigt, wobei der Querschnitt des Behandlungskopfes (5) derart beschaffen ist, dass der Behandlungkopf (5) durch die Mündung der Flasche (10) führbar ist und dass die Beschleunigungseinrichtung (6) die Ladungsträger derart beschleunigt, dass die aus dem Austrittsfenster (8) austretenden, Ladungsträger auf eine Innenwandung (15) der Flasche (10) richtbar sind und es sich bei den Ladungsträgern um Elektronen handelt, welche auf Energien von 100 keV bis 200 keV beschleunigt werden, wobei das Austrittsfenster aus Titan ist und eine Dicke aufweist, welche zwischen 5 µm und 20 µm liegt,
**dadurch gekennzeichnet, dass**
der Behandlungskopfquerschnitt einen Durchmesser aufweist, der unter 40 mm liegt,
wobei die Vorrichtung eine Einrichtung zum Kühlen des Austrittfensters mit einem gasförmigen Medium und eine Ablenkeinrichtung (7) zum Ablenken der Ladungsträger in einer radialen Richtung der Flasche (10) zur Flascheninnenwand vor Erreichen des Austrittsfensters (8) aufweist.

2. Vorrichtung (1) nach Anspruch 1,
**dadurch gekennzeichnet, dass**
das Austrittsfenster (8) und die Beschleunigungseinrichtung (6) innerhalb eines Außengehäuses (16) angeordnet sind und dieses Gehäuse (16) derart beschaffen ist, dass es durch die Mündung der Flasche (10) führbar ist.

3. Vorrichtung (1) nach einem der vorangegangenen Ansprüche,
**dadurch gekennzeichnet, dass**
die Vorrichtung (1) ein Innengehäuse (20) aufweist, innerhalb dessen die Beschleunigungseinrichtung (6) angeordnet ist und dass das Außengehäuse (16) dieses Innengehäuse (20) umgibt.

4. Vorrichtung (1) nach Anspruch 3,
**dadurch gekennzeichnet, dass**
zwischen dem Außengehäuse (16) und dem Innengehäuse (20) ein sich bis zu dem
Behandlungskopf (5) erstreckender Raum (22) gebildet wird, durch den ein Medium und insbesondere ein gasförmiges Medium führbar ist.

5. Vorrichtung (1) nach Anspruch 4,
**dadurch gekennzeichnet, dass**, der Raum (22) einen oder mehrere Kanäle (22a, 22b) aufweist, durch die jeweils ein gasförmiges oder flüssiges Medium geleitet werden kann.

6. Vorrichtung (1) nach einem der vorangegangenen Ansprüche,
**dadurch gekennzeichnet, dass**
der Behandlungskopf (5) gegenüber der Flasche (10) in einer Längsrichtung (L) der Flasche (10) und/oder eine radiale Richtung der Flasche bewegbar und/oder drehbar ist.

7. Anordnung (30) zum Behandeln von Flaschen mit einer Vorrichtung (1) nach einem der vorangegangenen Ansprüche, wobei die Anordnung (30) eine Vielzahl
von Vorrichtungen (1) zum Sterilisieren von Flaschen (10) nach einem der vorangegangenen Ansprüche 1 - 6 aufweist, wobei diese Vorrichtungen (1) entlang einer Kreisbahn angeordnet sind und an einem gemeinsamen Transportrad (3) angeordnet sind und wobei eine Vielzahl von Trägern (52) vorgesehen ist, an denen jeweils die Flaschen 10 angeordnet werden können.

8. Anordnung (30) nach Anspruch 7,
**dadurch gekennzeichnet, dass**
die Anordnung (30) eine Einrichtung (32) zum Befüllen von Flaschen (10) aufweist und die Vorrichtung (1) stromaufwärts bezüglich dieser Einrichtung (32) angeordnet ist.

9. Anordnung (30) nach Anspruch 7,
**dadurch gekennzeichnet, dass**
die Anordnung eine Verschiebeeinrichtung (36) aufweist, welche die Flaschen (10) in der Längsrichtung der Flaschen (10) gegenüber der Vorrichtung (1) verschiebt.

10. Anordnung nach einem der vorangegangenen Ansprüche 7 - 9,
**dadurch gekennzeichnet, dass**
die Anordnung eine weitere Sterilisationsvorrichtung (1b) zum Sterilisieren einer Außenwandung der Flaschen (10) aufweist.

11. Anordnung nach Anspruch 10,
**dadurch gekennzeichnet, dass**
die weitere Sterilisationsvorrichtung (1 b) stromaufwärts der Vorrichtungen (1) angeordnet ist.

12. Anordnung nach einem der vorangegangenen Ansprüche 10 - 11,
**dadurch gekennzeichnet, dass**
die weitere Sterilisationsvorrichtung (1 b) stationär angeordnet ist.

13. Anordnung nach einem der vorangegangenen Ansprüche 10 - 12,
**dadurch gekennzeichnet, dass**
die Anordnung ein Transportkarussell (42) aufweist, welches die Flaschen (10) an der weiteren Sterilisationsvorrichtung (1 b) vorbeiführt.

14. Verfahren zum Sterilisieren von Flaschen (10), wobei in einer Vorrichtung (1) zum Sterilisieren von Flaschen (10) Ladungsträger erzeugt und in Richtung eines Austrittfensters (8), welches in einem Behandlungskopf (5) angeordnet ist, beschleunigt werden, wobei der Behandlungskopf (5) der Vorrichtung (1) durch eine Mündung der Flasche (10) in das Innere der Flasche (10) eingeführt wird und ausgehend von dem Behandlungskopf (5) beschleunigte Ladungsträger direkt auf die Innenwandung der Flasche (10) gerichtet werden und die Flasche (10) relativ zu dem Behandlungskopf (5) bewegt wird, wobei bei den Ladungsträgern es sich um Elektronen handelt, welche auf Energien von 100 keV bis 200 keV beschleunigt werden, und wobei das Austrittsfenster aus Titan ist und eine Dicke aufweist, welche zwischen 5 µm und 20µm liegt,
**dadurch gekennzeichnet, dass**
der Behandlungskopfquerschnitt einen Durchmesser aufweist, der unter 40 mm liegt, und die Falschen 10 jeweils mittels einer Vielzahl von Trägern (52) entlang ihrer Längsrichtung bewegt werden, wobei ein gasförmiges Medium zum Kühlen des Austrittfensters dient und die Ladungsträger vor Erreichen des Austrittsfensters (8) in einer radialen Richtung der Flasche (10) zur Flascheninnenwand abgelenkt werden.

## Claims

1. Device (1) for sterilising bottles (10), comprising a mouth, with a treatment head (5) comprising an exit window (8) through which charge carriers can pass, comprising a charge carrier generation source which generates charge carriers, and comprising an acceleration device (6) which accelerates the charge carriers in the direction of the exit window (8), wherein the cross section of the treatment head (5) is dimensioned such that the treatment head (5) can be guided through the mouth of the bottle (10), and in that the acceleration device (6) accelerates the charge carriers in such a way that the charge carriers exiting from the exit window (8) can be aimed onto an inner wall (15) of the bottle (10) and the charge carriers are electrons which are accelerated to reach from 100 keV to 200 keV, wherein the exit window is made from titanium and has a thickness which lies between 5 µm and 20 µm,
**characterised in that**
the cross section of the treatment head has a diameter which lies below 40 mm, wherein the device comprises a means for cooling the exit window with a gaseous medium and a deflection device (7) for deviating the charge carriers in a radial direction of the bottle (10) towards the inner wall of the bottle before reaching the exit window (8).

2. Device (1) according to claim 1,
**characterised in that**
the exit window (8) and the acceleration device (6) are arranged within an outer housing (16) and this housing (16) is dimensioned such that it can be guided through the mouth of the bottle (10).

3. Device (1) according to one of the preceding claims,
**characterised in that**
the device (1) comprises an inner housing (20) within which the acceleration device (6) is arranged, and **in that** the outer housing (16) surrounds this inner housing (20).

4. Device (1) according to claim 3,
**characterised in that**
a chamber (22) which extends as far as the treatment head (5) is formed between the outer housing (16) and the inner housing (20), through which chamber a medium and in particular a gaseous medium can be guided.

5. Device (1) according to claim 4,
**characterised in that** the chamber (22) comprises one or more channels (22a, 22b), through which a gaseous or liquid medium can be passed in each case.

6. Device (1) according to at least one of the preceding claims,
**characterised in that**
the treatment head (5) can move and/or rotate relative to the bottle (10) in a longitudinal direction (L) of the bottle (10) and/or a radial direction of the bottle.

7. Arrangement (30) for treating bottles with a device (1) according to one of the preceding claims, wherein the arrangement (30) comprises a plurality of devices (1) for sterilising bottles (10) according to one of the preceding claims 1 to 6, wherein these devices (1) are arranged along a circular path and on a common transport wheel (3), a plurality of carriers (52) being provided on which respectively the bottles (10) can be arranged.

8. Arrangement (30) according to claim 7,
**characterised in that**
the arrangement (30) comprises a device (32) for filling bottles (10) and the device (1) is arranged upstream of this device (32).

9. Arrangement (30) according to claim 7,
**characterised in that**
the arrangement comprises a displacement device (36) which displaces the bottles in the longitudinal direction of the bottles (10) relative to the device (1).

10. Arrangement according to one of the preceding claims 7 to 9,
**characterised in that**
the arrangement comprises a further sterilising device (1 b) for sterilising an outer wall of the bottles (10).

11. Arrangement according to claim 10,
**characterised in that**
the further sterilising device (1 b) is arranged upstream of the devices (1).

12. Arrangement according to one of the preceding claims 10 to 11,
**characterised in that**
the further sterilising device (1 b) is arranged in a stationary manner.

13. Arrangement according to one of the preceding claims 10 to 12, **characterised in that**
the arrangement comprises a transport carousel (42) which conveys the bottles (10) past the further sterilising device (1 b).

14. Method for sterilising bottles (10), wherein charge carriers are generated in a device (1) for sterilising bottles (10) and are accelerated in the direction of an exit window (8) which is arranged in a treatment head (5), wherein the treatment head (5) of the device (1) is introduced through a mouth of the bottle (10) into the interior of the bottle (10) and accelerated charge carriers from the treatment head (5) are aimed directly onto the inner wall of the bottle (10) and the bottle (10) is moved relative to the treatment head (5) wherein the charge carriers are electrons which are accelerated to reach energy from 100 keV to 200 keV and wherein the exit window is made from titanium and has a thickness which lies between 5 µm and 20 µm,
**characterised in that**
the cross section of the treatment head has a diameter which lies below 40 mm and wherein the bottles (10) are respectively moved by means of a plurality of carriers (52) along the longitudinal direction thereof, wherein a gaseous medium serves for cooling the exit window and the charge carriers are deflected in a radial direction of the bottle towards the inner wall of the bottle before reaching the exit window (8).

## Revendications

1. Dispositif (1) de stérilisation de bouteilles (10), lesquelles présentent une embouchure, comprenant une tête de traitement (5) qui comporte une fenêtre de sortie (8) par laquelle des porteurs de charge peuvent passer, une source de génération de porteurs de charge qui génère des porteurs de charge, et un dispositif d'accélération (6), lequel accélère les porteurs de charge en direction de la fenêtre de sortie (8), la section transversale de la tête de traitement (5) étant d'une nature telle que la tête de traitement (5) peut être guidée à travers l'embouchure de la bouteille (10) et que le dispositif d'accélération (6) accélère les porteurs de charge de telle manière que les porteurs de charge sortant de la fenêtre de sortie (8) peuvent être orientés sur une paroi intérieure (15) de la bouteille (10) et les porteurs de charge étant des électrons, lesquels étant accélérés à des énergies allant de 100 keV à 200 keV, la fenêtre de sortie étant en titane et présentant une épaisseur comprise entre 5 µm et 20 µm,
**caractérisé en ce que**
la section transversale de la tête de traitement présente un diamètre inférieur à 40 mm,
le dispositif comprend un système de refroidissement de la fenêtre de sortie au moyen d'un milieu gazeux et un système de déviation (7) servant à dévier les porteurs de charge dans une direction radiale de la bouteille (10) vers la paroi intérieure de la bouteille avant d'atteindre la fenêtre de sortie (8).

2. Dispositif (1) selon la revendication 1,
**caractérisé en ce que**
la fenêtre de sortie (8) et le dispositif d'accélération (6) sont agencés à l'intérieur d'un boîtier extérieur (16) et ce boîtier (16) est d'une nature telle qu'il peut être guidé à travers l'embouchure de la bouteille (10).

3. Dispositif (1) selon l'une quelconque des revendications précédentes,
**caractérisé en ce que**
le dispositif (1) comprend un boîtier intérieur (20) à l'intérieur duquel le dispositif d'accélération (6) est agencé et **en ce que** le boîtier extérieur (16) entoure ce boîtier intérieur (20).

4. Dispositif (1) selon la revendication 3,
**caractérisé en ce que**
un espace (22) qui s'étend jusqu'à la tête de traitement (5), et à travers lequel un milieu et en particulier un milieu gazeux peut être guidé, est formé entre le boîtier extérieur (16) et le boîtier intérieur (20).

5. Dispositif (1) selon la revendication 4,
**caractérisé en ce que** l'espace (22) comprend un ou plusieurs canaux (22a, 22b) à travers lesquels un milieu gazeux ou liquide peut être dirigé dans chaque cas.

6. Dispositif (1) selon l'une quelconque des revendications précédentes,
**caractérisé en ce que**
la tête de traitement (5) peut être déplacée et/ou amenée en rotation par rapport à la bouteille (10) dans la direction longitudinale (L) de la bouteille (10) et/ou une direction radiale de la bouteille.

7. Ensemble (30) de traitement de bouteilles comprenant un dispositif (1) selon l'une quelconque des revendications précédentes, l'ensemble (30) comprenant une pluralité de dispositifs (1) de stérilisation de bouteilles (10) selon l'une quelconque des revendications précédentes 1 à 6, ces dispositifs (1) étant agencés le long d'une trajectoire circulaire et étant agencés sur une roue de transport (3) commune et une pluralité de supports (52) étant prévus, sur lesquels chaque bouteille (10) peut être placée.

8. Ensemble (30) selon la revendication 7,
**caractérisé en ce que**
l'ensemble (30) comprend un système (32) de remplissage de bouteilles (10) et le dispositif (1) est agencé en amont par rapport à ce système (32).

9. Ensemble (30) selon la revendication 7,
**caractérisé en ce que**
l'ensemble comprend un système de déplacement (36), lequel déplace les bouteilles (10) dans la direction longitudinale des bouteilles (10) par rapport au dispositif (1).

10. Ensemble selon l'une quelconque des revendications 7 à 9, **caractérisé en ce que**
l'ensemble comprend un autre dispositif de stérilisation (1 b) servant à stériliser une paroi extérieure des bouteilles (10).

11. Ensemble selon la revendication 10,
**caractérisé en ce que**
l'autre dispositif de stérilisation (1 b) est agencé en amont des dispositifs (1).

12. Ensemble selon l'une quelconque des revendications 10 à 11,
**caractérisé en ce que**
l'autre dispositif de stérilisation (1 b) est monté fixe.

13. Ensemble selon l'une quelconque des revendications 10 à 12,
**caractérisé en ce que**
l'ensemble comprend un carrousel de transport (42), lequel fait passer les bouteilles (10) devant l'autre dispositif de stérilisation (1 b).

14. Procédé de stérilisation de bouteilles (10), des porteurs de charge étant générés dans un dispositif (1) de stérilisation de bouteilles (10) et accélérés en direction d'une fenêtre de sortie (8), lesquels étant agencés dans une tête de traitement (5), la tête de traitement (5) du dispositif (1) étant introduite par une embouchure de la bouteille (10) à l'intérieur de la bouteille (10) et les porteurs de charge accélérés à partir de la tête de traitement (5) étant orientés directement vers la paroi intérieure de la bouteille (10) et la bouteille (10) étant déplacée par rapport à la tête de traitement (5), les porteurs de charge étant des électrons, lesquels étant accélérés à des énergies allant de 100 keV à 200 keV et la fenêtre de sortie étant en titane et présentant une épaisseur comprise entre 5 µm et 20 µm,
**caractérisé en ce que**
la section transversale de la tête de traitement présente un diamètre inférieur à 40 mm,
et les bouteilles (10) sont déplacées chacune au moyen d'une pluralité de supports (52) le long de leur direction longitudinale, un milieu gazeux servant à refroidir la fenêtre de sortie et les porteurs de charge étant déviés vers la paroi intérieure de la bouteille dans une direction radiale de la bouteille (10) avant d'atteindre la fenêtre de sortie (8).
